# EUROPEAN PATENT APPLICATION

(11) **EP 4 063 495 A1**
(43) Date of publication of application: **28.09.2022**
(21) Application number: 20890786.5
(22) Date of filing: 19.11.2020
(51) Int. Cl.: C12N 5/079, A61K 35/30, A61L 27/36, A61P 25/00, C12N 1/04, A61K 35/545

(54) **METHOD FOR FREEZING CELL AGGREGATES**

(30) Priority: 20.11.2019 JP 2019209931
(71) Applicant: Sumitomo Pharma Co., Ltd., Osaka 541-0045 (JP)
(72) Inventor: NAKAGAWA, Takashi, Suita-shi, Osaka 564-0053 (JP); TANAKA, Kazunari, Suita-shi, Osaka 564-0053 (JP)
(74) Representative: Mewburn Ellis LLP
(86) International application number: PCT/JP2020/043276
(87) International publication number: WO 2021/100830

(57) **Abstract**

Provided is a method for freezing a cell aggregate including neural cells. Provided is a method for freezing a cell aggregate including neural cells and having a three-dimensional structure, which comprises following steps (1) and (2): (1) soaking the cell aggregate including neural cells in a cryopreservation solution at 0°C to 30°C prior to freezing to prepare a cryopreservation solution-soaked cell aggregate; and (2) freezing the cell aggregate including neural cells in vapor phase of a liquid nitrogen container having a temperature of -150°C or less.

## Description

### TECHNICAL FIELD

The present application relates to a method for freezing a cell aggregate including neural cells.

### BACKGROUND ART

In recent years, development of drugs having cells as active ingredients has been promoted; for example, the cell therapy in which dopamine-producing neurons or progenitor cells thereof are induced from pluripotent stem cells (PSCs) and transplanted into the patient's mesencephalon is considered to be a promising treatment method for Parkinson's disease (Non Patent Literatures 1 to 3). For cell-based drugs, cryopreservation of a final product is an essential element for the dissemination of cell therapies (Patent Literatures 1 to 4). Unlike cell biology research, cryopreserved cells, when used in clinical practice, are transplanted immediately after thawing without recovery culture. Therefore, it is important for frozen cells to maintain their engraftment capacity, function or activity, and cell viability after thawing.

It has been suggested that transplantation of solid tissue induces a stronger immune response than transplantation of cell suspension because of the remaining donor blood vessels and antigen-presenting cells (Non Patent Literature 4). On the other hand, if the immune response is suppressed by isogeneic transplantation or an immunosuppressive agent, this problem is eliminated, and transplantation of ventral midbrain (VM) tissue shows dopamine-producing nerves having a higher survival rate and behavioral recovery than transplantation of cell suspension (Non Patent Literature 5). In addition, mechanical and enzymatic dissociation processes to obtain a cell suspension can alter cell properties to cause cell injury. Therefore, in clinical applications, it is desirable that a final product is formulated as a sphere rather than a cell suspension. However, spheres are more difficult to cryopreserve than single cells.

Slow cooling and vitrification methods are known as methods for freezing cells (Non Patent Literatures 6 to 8) .

The slow cooling method is a method in which cells are frozen together with a low concentration of cryoprotectant (CPA) (such as 10% dimethyl sulfoxide (DMSO)) at about 1 °C/min (Patent Literature 5, Non Patent Literatures 9 and 10). In the slow cooling method, ice formation begins first in the extracellular space, leading to the concentration of extracellular fluid. As a result, water is withdrawn from the cells by an osmotic gradient across the cell membrane. This dehydration of the cells avoids intracellular ice formation. However, if cells are excessively dehydrated, they are injured by the concentrated intracellular fluid and by the CPA in the cryopreservation solution.

On the other hand, the vitrification method is an ultra-fast cooling method in which cells are transferred to liquid nitrogen immediately after the addition of a high concentration of cryoprotectant (e.g., DMSO, acetamide, or ethylene glycol) and are frozen in an amorphous state at once (Patent Literature 6, Non Patent Literature 11). In other words, it is a method of minimizing the growth of ice crystals by solidifying a solvent inside and outside cells in the glass state. The vitrification method requires strict time control; in the case of a cell aggregate, it takes time for a cryopreservation solution to permeate into the cell aggregate unlike in the case of single cells dispersed in a solvent, and therefore vitrification effect on the cryopreservation solution may not be sufficiently obtained. Accordingly, its application to clinical cell production has been technically difficult (Non Patent Literature 12).

As stated above, because of the need for precise control of ice formation and cell dehydration, no clinical cryopreservation method for a sphere has been established.

In addition, though Non Patent Literatures 13 discloses that mycoplasma contamination can be prevented by freezing cultured cells in the vapor phase using a vapor-phase liquid nitrogen storage container that can be stored at -190°C, freezing a cell aggregate in the vapor phase of liquid nitrogen has been completely unknown.

### Citation List

### Patent Literature

[Patent Literature 1]
   JP2017-104061A
[Patent Literature 2]
   WO2017/159862
[Patent Literature 3]

   JP2015-521469A
[Patent Literature 4]
   JP2008-501320A
[Patent Literature 5]
   JP2011-103885A
[Patent Literature 6]
   JP2013-110988A

### Non Patent Literature

[Non Patent Literature 1]
   Doi et al., Stem Cell Reports, 2(3), 337-350, 2014
[Non Patent Literature 2]
   Sundberg et al., Stem Cells 31, 1548-1562, 2013
[Non Patent Literature 3]
   Nolbrant et al., Nature Protocols, 12(9), 1962-1979, 2017
[Non Patent Literature 4]
   Redmond et al., Neurobiology of Disease, 29(1), 103-116, 2008
[Non Patent Literature 5]
   Fricker et al., PLoS ONE, 7(10), e47169, 2012
[Non Patent Literature 6]
   Chong et al., Stem Cells, 27(1), 29-39, 2009
[Non Patent Literature 7]
   Smith et al., Fertility and Sterility, 94(6), 2088-2095, 2010
[Non Patent Literature 8]
   Jang et al., Integrative Medicine Research, 6(1), 12-18, 2017
[Non Patent Literature 9]
   Schwartz et al., Journal of Neuroscience Research, 74(6), 838-851, 2003
[Non Patent Literature 10]
   Woods et al., Cryobiology, 59(2), 150-157, 2009
[Non Patent Literature 11]
   Fahy and Wowk., Methods in Molecular Biology (Methods and Protocols), vol 1257. Springer, New York, NY, 2015
[Non Patent Literature 12]
   Nagano et al., Biomedical Research, 28(3), 153-160, 2007
[Non Patent Literature 13]
   Tiss Cult Res Commun 26: 165-170 (2007)

### SUMMARY OF INVENTION

### TECHNICAL PROBLEM

An object of the present application is to provide a method for freezing a cell aggregate including neural cells.

### SOLUTION TO PROBLEM

As a result of intensive studies, the present inventors have found a method for freezing a cell aggregate including neural cells and have completed the present invention. More specifically, the present invention relates to the following:
[1] A method for freezing a cell aggregate including neural cells and having a three-dimensional structure, which comprises following steps (1) and (2):
   (1) soaking the cell aggregate including neural cells in a cryopreservation solution at 0°C to 30°C prior to freezing to prepare a cryopreservation solution-soaked cell aggregate; and
   (2) freezing the cryopreservation solution-soaked cell aggregate in vapor phase of a liquid nitrogen container having a temperature of -150°C or less.
[2] The method according to [1], wherein the cell aggregate is soaked in the cryopreservation solution for 15 minutes to 360 minutes in step (1).
[3] The method according to [1] or [2], wherein a proportion of volume of the cell aggregate and the cryopreservation solution contained in the liquid nitrogen container to volume of the vapor phase of the liquid nitrogen container is 5% or less.
[4] The method according to any one of [1] to [3], wherein a filling density of the cell aggregate relative to the preservation solution is 50 to 500 cell aggregates/mL.
[5] The method according to [4], wherein size of the container containing the cell aggregate and the cryopreservation solution is 0.5 to 5 mL.
[6] The method according to any one of [1] to [5], wherein the cell aggregate including neural cells is a cell aggregate including neural cells derived from pluripotent stem cells.
[7] The method according to any one of [1] to [6], wherein the cell aggregate including neural cells comprises cells that are positive for at least one of FOXA2, TH, and NURR1.
[8] The method according to [7], wherein the cell aggregate including neural cells comprises cells positive for FOXA2 and LMX1A.
[9] The method according to [7], wherein the cell aggregate including neural cells comprises cells positive for FOXA2, TH, and NURR1.
[10] The method according to any one of [1] to [9], wherein the neural cells are dopamine-producing neurons or progenitor cells thereof.
[11] The method according to any one of [1] to [10], wherein the cell aggregate includes 60% or more of dopamine-producing neuron progenitor cells.
[12] The method according to any one of [1] to [11], wherein the cell aggregate including neural cells is a cell aggregate having an equivalent spherical diameter of 150 µm to 1000 µm.
[13] The method according to [12], wherein the cell aggregate includes 500 to 150000 cells.
[14] The method according to any one of [1] to [13], wherein number of cells contained in the cryopreservation solution is 80000 to 5000000 cells/mL.
[15] A method for preserving a cell aggregate including neural cells for a long-term, wherein the method comprises storing a container containing the cell aggregate obtained by the method according to any one of [1] to [14] in a vapor phase or a liquid phase of the liquid nitrogen container.
[16] A composition for transplantation, wherein the composition comprises, as an active ingredient, the cell aggregate obtained by the method according to any one of [1] to [14] .
[17] The composition for transplantation according to [16], wherein the composition comprises: a cell aggregate including 60% or more of dopamine-producing neuron progenitor cells and having an equivalent spherical diameter of 150 µm to 1000 µm; and a cryopreservation solution, and wherein the composition can be used without recovery culture after thawing.
[18] The composition for transplantation according to [16] or [17], wherein number of cells in the composition is 80000 to 5000000 cells/mL.
[19] The composition for transplantation according to any one of [16] to [18], wherein the composition comprises 10 to 500 cell aggregates/mL.
[20] The composition for transplantation according to any one of [16] to [19], wherein the cell aggregate and the cryopreservation solution are filled in a 0.5 mL to 15 mL container.
[21] A method for producing a composition for transplantation comprising dopamine-producing neuron progenitor cells as an active ingredient, which comprises:
   freezing a cell aggregate by the method according to any one of [1] to [14], wherein number of cells in the composition is 80000 to 5000000 cells/mL, and the cell aggregate comprises 60% or more of the dopamine-producing neuron progenitor cells and has an equivalent spherical diameter of 150 µm to 1000 µm.
[22] A method for producing the composition for transplantation according to [21], wherein the cell aggregate and the cryopreservation solution are filled in a 0.5 mL to 15 mL container.
[23] A method for treating a disease requiring regeneration of a dopamine nerve, which comprises following steps of:
   (1) thawing the composition for transplantation according to any one of [16] to [20] at 30°C to 40°C; and
   (2) transplanting the composition for transplantation obtained in (1) into a corpus striatum region of a patient.
[24] The method according to [23], wherein the cryopreservation solution is replaced with a dosing vehicle without culture after the thawing in (1) to perform step (2).

### EFFECTS OF THE INVENTION

The present application provides a method for freezing a cell aggregate including neural cells. The cell aggregate of neural cells frozen by the method of the present application exhibits high cell viability and maintains functional properties such as neurite extension properties. Further, when the present invention is used, the frozen cell aggregate can be preserved without transferring it to a preservation apparatus. Accordingly, temporary temperature rise due to the transfer to the preservation apparatus can be avoided, causing no unwanted damage to the cell aggregate. Even in the case of preservation at a temperature lower than the vapor phase of liquid nitrogen, the cell aggregate can be directly soaked in liquid nitrogen without taking it out of the container, thereby reducing number of steps and improving workability for an industrial production method.

### BRIEF DESCRIPTION OF DRAWINGS

[Figure 1] Cell viability when the cell aggregates frozen in a liquid nitrogen cryopreservation container (vapor phase preservation type), a -80°C deep freezer or a -150°C deep freezer were thawed.
[Figure 2] The effect of pre-soaking time in a cryopreservation solution before freezing on cell viability of the cell aggregates after freezing and thawing.
[Figure 3] The effect of pre-soaking time in a cryopreservation solution before freezing on a neurite extension capacity of the cell aggregates after freezing and thawing.
[Figure 4] The effect of volume of the vapor phase of a liquid nitrogen container on cell viability of cell aggregates after freeze and thawing.
[Figure 5] The effect of volume of the vapor phase of a liquid nitrogen container on a neurite extension capacity of the cell aggregates after freezing and thawing.
[Figure 6] The effect of number of the cell aggregates filled in a sample container on cell viability of the cell aggregates after freezing and thawing.
[Figure 7] The effect of number of the cell aggregates filled in a sample container on a neurite extension capacity of the cell aggregates after freezing and thawing.
[Figure 8] The effect of volume of the filled solution on cell viability of the cell aggregates after freezing and thawing.
[Figure 9] The effect of volume of the filled solution on a neurite extension capacity of the cell aggregates after freezing and thawing.
[Figure 10] The effect of an equivalent spherical diameter of the cell aggregates on cell viability of the cell aggregates after freezing and thawing.
[Figure 11] The effect of an equivalent spherical diameter of the cell aggregates on a neurite extension capacity of the cell aggregates after freezing and thawing.
[Figure 12] Cell viability after thawing of the cell aggregates frozen in the vapor or liquid phase of liquid nitrogen.

### DESCRIPTION OF EMBODIMENTS

In the present specification and claims, when a numerical value is accompanied by the term "about," it is intended to include a range of ± 10% of the value. For example, "about 20" shall include "18 to 22". A numerical range includes all numerical values between the two endpoints and the numerical values at both endpoints. The term "about" for a range applies to both endpoints of the range. Thus, for example, "about 20 to 30" shall include "18 to 33".

### [Neural cells]

The present application provides a method for freezing a cell aggregate including neural cells and having a three-dimensional structure.

The neural cells include neuronal cells or neurons, progenitor cells of the neurons, i.e., neural progenitor cells or neural precursor cells.

The neural cells may be neural cells derived from any site such as neural cells of the central nervous system, or neural cells of the peripheral nervous system such as somatic neural cells of motor nerves or sensory systems or neural cells of autonomic nerves; examples of the neural cells include nerves (neurons), neural crest-derived cells, glia cells such as oligodendrocytes or astrocytes, and stem cells or progenitor cells thereof. Examples of the neural cell include a cell expressing a neural cell marker. Examples of the neural cell marker include, but are not limited to, NCAM, βIII-Tubulin (TUJ1), tyrosine hydroxylase (TH), serotonin, nestin, MAP2, MAP2AB, NEUN, GABA, glutamate, CHAT, SOX1, BF1, EMX1, VGLUT1, PAX, NKX, GSH, Telencephalin, GLUR1, CAMKII, CTIP2, TBR1, Reelin, TBR1, BRN2, OTX2, LMX1A, LMX1B, EN1, NURR1, PITX3, DAT, GIRK2, and TH. The neural cell can be identified by the expression of one or more neural cell markers. In the present specification, examples of the neural cell include a cell expressing one or more, two or more, or three or more of the above neural cell markers.

Neural cells of the central nervous system can be classified according to differences in sites where the neural cells nervous systems are present. That is, examples of the neural cells of the central nervous system include neurons derived from prosencephalon, telencephalon, diencephalon, cerebral, hypothalamus, mesencephalon, metencephalon, mesencephalon-metencephalon boundary region, cerebellum, retina, pituitary gland, or spinal cord, and progenitor cells thereof.

The neurons derived from prosencephalon are neurons that are present in prosencephalon tissue (i.e., telencephalon, cerebral, hippocampus or choroid plexus, diencephalon, hypothalamus, etc.). The neurons of prosencephalon can be identified by the expression of a prosencephalon neuron marker. Examples of the prosencephalon neuron marker include OTX1 (prosencephalon), BF1 (also referred to as FOXG1) or SIX3 (also serving as a marker for telencephalon or cerebral). In the present specification, examples of the neural cell include a cell expressing one or more, two or more, or three or more of the above prosencephalon neuron markers or the markers for telencephalon or cerebral.

Examples of the neuron derived from cerebral include dorsal cells (e.g., cerebral cortex cells, Cajal-Retzius cells, hippocampus neurons) and ventral cells (e.g., basal ganglia). Examples of the ventral cerebral neuron marker include basal ganglia neuron markers (e.g., GSH2, MASH1, NKX2.1, NOZ1). Examples of the dorsal cerebral neuron marker include cerebral cortex neuron markers (e.g., PAX6, EMX1, TBR1). In the present specification, examples of the neural cell include a cell expressing one or more, two or more, or three or more of the above cerebral neuron markers, the basal ganglia neuron markers or the cerebral cortex neuron markers.

Examples of the neural cell derived from mesencephalon include neural progenitor cells derived from ventral mesencephalon, dopamine-producing neurons (also referred to as dopaminergic neurons) and dopamine-producing neuron progenitor cells (also referred to as dopaminergic neuron progenitor cells or dopaminergic progenitors). Examples of a mesencephalon-derived neural cell marker include FOXA2, EN2, and TUJ1. Examples of a FOXA2-positive and TUJ1-positive neural cell include dopamine-producing neuron progenitor cells and dopamine-producing neurons. The dopamine-producing neurons can be identified by being FOXA2-positive, NURR1-positive, and TH-positive.

The dopamine-producing neuron progenitor cells can be identified by being FOXA2-positive and LMX1A-positive. The dopamine-producing neuron progenitor cells more preferably contain cells that are positive for one or more of OTX2, LMX1A, LMX1B, CORIN, SHH, AADC, βIII-Tubulin, EN1, NURR1, PITX3, DAT, GIRK2, and TH. In the present specification, a cell aggregate including dopamine-producing neuron progenitor cells, unless otherwise specified, may comprise dopamine-producing neurons or dopaminergic neurons.

In the present specification, examples of the neural cell include a cell expressing one or more, two or more, or three or more of the mesencephalon-derived neural cell markers, the dopamine-producing neuron progenitor cell markers or the dopamine-producing neuron markers.

In the present specification, examples of the dopamine-producing neuron progenitor cells include cells expressing FOXA2 and/or LMX1A (FOXA2-positive and/or LMX1A-positive), preferably cells expressing one or more, two or more, or three or more selected from the group consisting of OTX2, LMX1B, EN1, CORIN, SHH, AADC, and βIII-Tubulin in addition to FOXA2 and LMXA1.

In the present specification, examples of the dopamine-producing neurons (dopaminergic neurons) include cells expressing TH and/or NURR1 (TH-positive and/or NURR1-positive), preferably cells expressing one or more, two or more, or three or more selected from the group consisting of FOXA2, AADC, DAT, and GIRK2 in addition to TH and NURR1.

Examples of the neurons derived from the mesencephalon-metencephalon boundary region include neurons that are present in the cerebellum, cerebellar plate tissue, ventricular zone, rhombic lip, etc. Examples of a mesencephalon-metencephalon boundary region marker include EN2 (mesencephalon), GBX2 (metencephalon), N-Cadherin (neural progenitor cells in the mesencephalon-metencephalon boundary region). Examples of a cerebellar neural progenitor cell marker include KIRREL2, PTF1A, and SOX2, which are GABAergic progenitor cell markers, and ATOH1 and BARHL1, which are cerebellar granule cell progenitor cell markers. In the present specification, examples of the neural cell include a cell expressing one or more, two or more, or three or more of the above mesencephalon-metencephalon boundary region markers, the cerebellar neural progenitor cell markers, the GABAergic progenitor cell markers, or the cerebellar granule cell progenitor cell markers.

Examples of a neural cell derived from a retina include photoreceptor cells, photoreceptor progenitor cells, retinal pigment epithelium cells, and cornea cells.

The neural cells can also be classified according to differences in neurotransmitters produced (secreted); examples thereof include dopamine-producing neurons, dopamine-producing neuron progenitor cells, GABAergic neurons, GABAergic progenitor cells, cholinergic neurons, cholinergic progenitor cells, serotonergic neurons, serotonergic progenitor cells, glutamatergic neurons, glutamatergic progenitor cells, noradrenergic neurons, noradrenergic progenitor cells, adrenergic neurons, and adrenergic progenitor cells.

Examples of the neural cells of motor nerves or sensory systems include cholinergic neurons and progenitor cells thereof.

Examples of the neural cells of autonomic nerves include cholinergic neurons, adrenergic neurons, and progenitor cells thereof.

Preferred examples of the neural cells in the present specification include dopamine-producing neurons (dopaminergic neurons), and dopamine-producing neuron progenitor cells (dopaminergic neuron progenitor cells).

Neural cells derived from a living body are cells isolated from a mammal such as humans, and examples of the cells isolated from human brain tissue include cells contained in the fetal mesencephalon tissue as described in Nature Neuroscience, 2, 1137 (1999) or N. Engl. J. Med.; 344: 710-9 (2001).

The neural cells may also be cells obtained by inducing differentiation from pluripotent stem cells such as embryonic stem cells (ES cells) and iPS cells. Examples of a method for inducing differentiation from pluripotent stem cells into neural cells include the methods described in Non Patent Literatures 3 and 4 and WO2015/034012 (dopaminergic neuron progenitor cells), WO2009/148170 (cerebral neural cells and the like), WO2013/065763, WO2016/013669 or WO2017/126551 (hypophysial or subthalamic neural cells), WO2016/039317 (cerebellar neural cells), WO2015/076388 (telencephalic neural cells), Numasawa-Kuroiwa, Y et al., Stem Cell Reports, 2: 648-661 (2014) (neural progenitor cells), Qiu, L et al., Stem Cells Transl Med. 6 (9): 1803-1814 (2017) (dopamine-producing neuron progenitor cells).

The neural cells may be cells obtained by inducing differentiation from multipotent stem cells such as mesenchymal stem cells (MSCs). Examples of a method for inducing differentiation from mesenchymal stem cells into neural cells include the method described in J Chem Neuroanat. 96: 126-133 (2019).

### [Pluripotent stem cell]

A pluripotent stem cell refers to a stem cell that has both pluripotency that enables differentiation into almost all cells existing in a living body and proliferative capacity. Pluripotent stem cells can be derived from fertilized ova, cloned embryos, germ stem cells, interstitial stem cells, or somatic cells. Examples of the pluripotent stem cell include, but are not particularly limited to, embryonic stem (ES) cells, nuclear transfer embryonic stem (ntES) cells derived from cloned embryos, spermatogonial stem cells (GS cells), embryonic germ cells (EG cells), induced pluripotent stem (iPS) cells, pluripotent stem cells derived from cultured fibroblasts and bone marrow stem cells (Muse cells). The pluripotent stem cells may be ES cells, ntES cells, or iPS cells. The pluripotent stem cells may be iPS cells in view of ethical considerations. Note that the embryonic stem cells are established from embryos within 14 days of fertilization.

Embryonic stem cells were first established in 1981 and have been applied to the production of knockout mice since 1989. Human embryonic stem cells were established in 1998 and are being used in regenerative medicine. Embryonic stem cells can be produced by culturing an inner cell mass on feeder cells or in medium containing leukemia inhibitory factor (LIF). Methods for producing embryonic stem cells are described, for example, in WO96/22362, WO02/101057, US5,843,780, US6,200,806, US6,280,718, etc. Embryonic stem cells can be obtained from a given institution or purchased commercially. For example, human embryonic stem cells KhES-1, KhES-2, and KhES-3 are available from Institute for Frontier Life and Medical Sciences, Kyoto University. Human embryonic stem cell line Rx::GFP (derived from the KhES-1 line) is available from Institute of Physical and Chemical Research. Mouse embryonic stem cell lines, EB5 and D3, are available from Institute of Physical and Chemical Research and ATCC, respectively.

A nuclear transfer embryonic stem cell (ntES cell), which is one type of embryonic stem cell, can be established from a cloned embryo created by transferring the nucleus of a somatic cell into an ovum from which the nucleus has been removed.

EG cells can be produced by culturing primordial germ cells in medium containing mSCF, LIF, and bFGF (Cell, 70: 841-847, 1992).

The term "induced pluripotent stem cell" as used herein refers to a cell obtained by reprogramming a somatic cell to have pluripotency induced using a known method. Specific examples thereof include cells obtained by reprogramming differentiated somatic cells such as fibroblasts or peripheral blood mononuclear cells by expression of any combination of a plurality of genes selected from a reprogramming gene group including OCT3/4, SOX2, KLF4, MYC (c-MYC, N-MYC, L-MYC), GLIS1, NANOG, SALL4, LIN28, ESRRB to induce pluripotency. Preferred examples of the combination of reprogramming factors include (1) OCT3/4, SOX2, KLF4, and MYC (c-MYC or L-MYC), (2) OCT3/4, SOX2, KLF4, LIN28, and L-MYC (Stem Cells, 2013; 31: 458 to 466), and (3) OCT3/4, SOX2, NANOG, and LIN28 (Science 2007; 318: 1917 to 1920).

Induced pluripotent stem cells were established with mouse cells by Yamanaka et al. in 2006 (Cell, 2006, 126 (4), pp. 663 to 676). Induced pluripotent stem cells were also established with human fibroblasts in 2007 and have pluripotency and replication competence similar to embryonic stem cells (Cell, 2007, 131 (5), pp. 861 to 872; Science, 2007, 318 (5858), pp. 1917 to 1920; Nat. Biotechnol., 2008, 26 (1), pp. 101 to 106).

Induced pluripotent stem cells can also be produced by a method of deriving induced pluripotent stem cells from somatic cells through the addition of a compound or the like, in addition to a method of producing induced pluripotent stem cells by reprogramming directly through gene expression (Science, 2013, 341, pp. 651 to 654).

It is also possible to obtain an established induced pluripotent stem cell line; for example, human induced pluripotent stem cell lines such as 201B7 cells, 201B7-Ff cells, 253G1 cells, 253G4 cells, 1201C1 cells, 1205D1 cells, 1210B2 cells, 1231A3 cells, etc. established at Kyoto University are available from Kyoto University. For example, Ff-I01 cells, Ff-I01s04 cells, QHJ-I01 and Ff-I14 cells established at Kyoto University are available from Kyoto University as the established induced pluripotent stem cell line.

Examples of somatic cells used in the production of induced pluripotent stem cells include, but are not particularly limited, tissue-derived fibroblasts, erythroid cells (e.g., peripheral blood mononuclear cells (PBMCs), T cells), hepatocytes, pancreatic cells, enterocytes, and smooth muscle myocytes.

For reprogramming through expression of several genes in the production of induced pluripotent stem cells, the means for expressing the genes is not particularly limited. Examples of the above-mentioned means include infection with a viral vector (e.g., retroviral vectors, lentiviral vectors, Sendai viral vectors, adenoviral vectors, or adeno-associated virus vectors), gene transfer (e.g., calcium phosphate transfection, lipofection, RetroNectin method, or electroporation) using a plasmid vector (e.g., plasmid vectors or episomal vectors), gene transfer (e.g., calcium phosphate transfection, lipofection, or electroporation) using an RNA vector, and direct injection (e.g., needle-based method, lipofection, or electroporation) of a protein.

Induced pluripotent stem cells can be produced in the presence of feeder cells or in the absence of feeder cells (feeder-free). In the production of induced pluripotent stem cells in the presence of feeder cells, the induced pluripotent stem cells can be produced in the presence of an undifferentiated-state maintenance factor by a known method. Medium used to produce induced pluripotent stem cells in the absence of feeder cells is not particularly limited and can be any known maintenance medium for embryonic stem cells and/or induced pluripotent stem cells, or any medium for establishing induced pluripotent stem cells in a feeder-free manner. Examples of the medium for establishing induced pluripotent stem cells in a feeder-free manner include feeder-free media such as Essential 8 medium (E8 medium), Essential 6 medium, TeSR medium, mTeSR medium, mTeSR-E8 medium, Stabilized Essential 8 medium, and StemFit medium. In the production of induced pluripotent stem cells, for example, four factors, OCT3/4, SOX2, KLF4, and MYC (L-MYC or c-MYC) can be transferred into somatic cells in a feeder-free manner using Sendai viral vectors to prepare the induced pluripotent stem cells.

Pluripotent stem cells used in the present invention are mammalian pluripotent stem cells, preferably rodent (e.g., mouse or rat) or primate (e.g., human or monkey) pluripotent stem cells, more preferably human or mouse pluripotent stem cells, even more preferably human induced pluripotent stem cells (iPS cells) or human embryonic stem cells (ES cells).

### [Cell aggregate]

The cell aggregate "having a three-dimensional structure" herein refers to a cell aggregate (cell aggregate or sphere) which is a three-dimensional cell population formed by cultured cells adhering to each other through, for example, a suspension culture or a 3D culture. A cell aggregate of neural cells is also referred to as a neurosphere. The shape of the cell aggregate is not particularly limited and may be spherical or non-spherical. The cell aggregate in the present specification is preferably a cell aggregate having a three-dimensional shape similar to a sphere. The three-dimensional shape similar to a sphere is a shape having a three-dimensional structure and shows, for example, a circular shape or an elliptical shape when projected onto a two-dimensional plane.

The cell aggregate including neural cells and having a three-dimensional structure has no particular restrictions on its size but usually has an equivalent spherical diameter of 150 µm to 1000 µm, and for example, 200 µm to 800 µm or 300 µm to 500 µm in one embodiment. The cell aggregate including neural cells and having a three dimensional structure usually includes 500 to 150000 cells, and in one embodiment, for example, 1000 to 100000 cells, 1000 to 70000 cells, or 3000 to 30000 cells.

The cell aggregate including neural cells may comprise other cells together with the neural cells. Examples of such a cell aggregate include a cell aggregate including 60% or more, 70% or more, 80% or more, and more preferably 90% or more of neural cells.

In one embodiment, the cell aggregate including neural cells may comprise 60% or more, 70% or more, or 80% or more of dopamine-producing neuron progenitor cells and/or dopamine-producing neurons. That is, the cell aggregate including neural cells may comprise neural cells expressing one or more markers selected from FOXA2, LMX1A, LMX1B, NURR1, and TH in an amount of 60% or more, 70% or more, or 80% or more.

In one embodiment, the cell aggregate including neural cells comprises 40% or more, 60% or more, 70% or more, 80% or more, 85% or more, or 90% or more of dopamine-producing neuron progenitor cells.

In one embodiment, the cell aggregate including neural cells comprises cells expressing one or more, two or more, or three or more markers for dopamine-producing neuron progenitor cells in an amount of 40% or more, 60% or more, 70% or more, 80% or more, 85% or more, or 90% or more.

In one embodiment, the cell aggregate including neural cells comprises cells positive for FOXA2 and LMX1A in an amount of 40% or more, 60% or more, 70% or more, 80% or more, 85% or more, or 90% or more. In one embodiment, the cell aggregate further comprises cells positive for TH and NURR1 in an amount of 40% or less.

In one embodiment, the cell aggregate including neural cells may comprise cells positive for FOXA2, TH, and NURR1 in an amount of 0% or more, 10% or more, or 20% or more.

In one embodiment, the cell aggregate including dopamine-producing neuron progenitor cells may comprise NURR1-positive cells in an amount of 60% or less, 50% or less, 40% or less, 5 to 50%, 5 to 40%, or 5 to 20%.

In one embodiment, the cell aggregate including dopamine-producing neuron progenitor cells and/or dopamine-producing neurons may comprise TH-positive cells in an amount of 30% or less, 20% or less, 1 to 30%, 5 to 30%, 1 to 20%, 5 to 20%, or 5 to 15%.

In one embodiment, the cell aggregate including dopamine-producing neuron progenitor cells and/or dopamine-producing neurons may comprise KI67-positive cells in an amount of 30% or less, 1 to 25%, 1 to 20%, or 5 to 20%.

In one embodiment, the cell aggregate including dopamine-producing neuron progenitor cells and/or dopamine-producing neurons may comprise SOX1-positive cells in an amount of 20% or less, 10% or less, 5% or less, or 1% or less.

In one embodiment, the cell aggregate including dopamine-producing neuron progenitor cells and/or dopamine-producing neurons may comprise PAX6-positive cells in an amount of 5% or less, 2% or less, 1% or less, or 0.5% or less.

In one embodiment, the cell aggregate including dopamine-producing neuron progenitor cells and/or dopamine-producing neurons further comprises cells positive for TH and NURR1 in an amount of 20% or less, specifically 1% to 20%, more specifically 5% to 15%.

In one embodiment, the cell aggregate including dopamine-producing neuron progenitor cells and/or dopamine-producing neurons comprises cells positive for FOXA2 and LMX1A in an amount of 50% or more, preferably 60% or more, 70% or more, or 80% or more, and cells positive for TH and NURR1 in an amount of 20% or less, 1% to 20%, more specifically 5% to 15%.

In one embodiment, in the cell aggregate including dopamine-producing neuron progenitor cells and/or dopamine-producing neurons, SOX1-positive cells are 10% or less, preferably 7% or less, more preferably 3% or less, and PAX6-positive cells are 5% or less, preferably 4% or less, more preferably 2% or less.

In one embodiment, the cell aggregate including dopamine-producing neuron progenitor cells and/or dopamine-producing neurons comprises cells positive for FOXA2 and LMX1A in an amount of 60% or more and cells positive for TH and NURR1 in an amount of 1% to 20%, and SOX1-positive cells are 10% or less, preferably 7% or less, more preferably 3% or less, and PAX6-positive cells are 5% or less, preferably 4% or less, more preferably 2% or less.

In one embodiment, the cell aggregate including dopamine-producing neuron progenitor cells and/or dopamine-producing neurons comprises cells positive for FOXA2 and LMX1A in an amount of 60% or more of the total cells and may comprise cells positive for TH and NURR1 in an amount of 20% or less, 1 to 20%, or 5 to 15% of the total cells.

In one embodiment, the above cell aggregate including dopamine-producing neuron progenitor cells and/or dopamine-producing neurons is a cell aggregate having an equivalent spherical diameter of 150 µm to 1000 µm.

In one embodiment, the above cell aggregate including dopamine-producing neuron progenitor cells and/or dopamine-producing neurons comprises cells positive for FOXA2 and LMX1A in an amount of 60% or more and cells positive for NURR1 and TH in an amount of 1% to 20% and has an equivalent spherical diameter of 150 µm to 1000 µm.

### [Freezing method]

The method of the present application comprises the step of (1) soaking a cell aggregate including neural cells and having a three-dimensional structure in a cryopreservation solution at 0°C to 30°C prior to freezing to prepare a cryopreservation solution-soaked cell aggregate.

In the present application, a cryopreservation solution refers to an aqueous solution comprising a cryoprotectant. The cryoprotectant refers to a substance that have a high affinity with water molecules and are highly effective in inhibiting the growth of ice crystals in the cryopreservation solution. Cryoprotectants include, for example, dimethyl sulfoxide (DMSO), ethylene glycol (EG), propylene glycol (PG), 1,2-propanediol (1,2-PD), 1, 3-propanediol (1,3-PD), butylene glycol (BG), isoprene glycol (IPG), dipropylene glycol (DPG), and glycerin. In the present application, the cryoprotectant is preferably dimethyl sulfoxide, glycerin, and/or propylene glycol. A concentration of the cryoprotectant in the cryopreservation solution is usually about 2 to 12%, preferably about 5 to 12%, more preferably about 7 to 12%, even more preferably about 7 to 10%, still even more preferably about 10%, when dimethyl sulfoxide, glycerin, and/or propylene glycol is used as the cryoprotectant.

As the aqueous solution, for example, physiological saline, a buffered solution such as PBS, EBSS, or HBSS, a medium for culturing cells or tissues such as DMEM, GMEM, or RPMI, serum, a serum substitute, or a mixture thereof can be used.

As the cryopreservation solution, a commercially available cryopreservation solution comprising dimethyl sulfoxide (DMSO), glycerin, and/or propylene glycol as a major component can be used. Specific examples of the cryopreservation solution include commercially available cryopreservation solutions such as STEM-CELL BANKER (SCB; ZENOAQ), STEM-CELL BANKER DMSO free (SCB DMSO free; ZENOAQ), Bambanker hRM (BBK; NIPPON Genetics), Bambanker DMSO Free (BBK DMSO Free; NIPPON Genetics), CryoStor CS5 (CS5; BioLife Solutions), CryoStor CS10 (CS10; BioLife Solutions), and Synth-a- Freeze (SaF; Thermo Fisher Scientific). For example, it is desirable to use a cryopreservation solution (such as STEM-CELL BANKER, Bambanker hRM, CryoStor CS10, or Synth-a-Freeze) comprising 7 to 12%, preferably about 10% of dimethyl sulfoxide, glycerin, and/or propylene glycol.

More preferably, Bambanker hRM can be used.

In the present specification, when the cell aggregate is frozen, number of cells relative to the cryopreservation solution (cell packing density) is 80000 to 5000000 cells/mL, 100000 to 4000000 cells/mL or 200000 to 2000000 cells/mL, 300000 to 1000000 cells/mL.

In the present specification, when the cell aggregate is frozen, the cell aggregate has an equivalent spherical diameter of 150 to 1000 µm, 150 µm to 600 µm, or 300 µm to 500 µm.

In the present specification, the cell aggregate and the preservation solution have volume of 0.25 mL to 2 mL, 0.5 mL to 1.5 mL, or 0.5 mL to 1 mL.

In the present specification, the cell aggregate and the preservation solution may be packed in a 0.5 mL to 15 mL, 1 mL to 5 mL, or 1 mL to 2 mL container.

The freezing point of the cryopreservation solution in the present application is not particularly limited but is usually about -1°C to -10°C, preferably -3°C to -10°C, more preferably -3°C to -6°C, even more preferably about - 5°C.

The temperature at which the cell aggregate including neural cells is soaked in the cryopreservation solution is usually from 0°C to 30°C, preferably from 0°C to 20°C, more preferably from 0°C to 10°C, and even more preferably from 0°C to 5°C.

The period for which the cell aggregate including neural cells is soaked in the cryopreservation solution is usually 5 minutes to 360 minutes, 5 minutes to 240 minutes, 5 minutes to 180 minutes, 5 minutes to 120 minutes, preferably 5 minutes to 60 minutes, 15 minutes to 360 minutes, 15 minutes to 240 minutes, 15 minutes to 180 minutes, 15 minutes to 150 minutes, preferably 15 minutes to 120 minutes and more preferably 15 minutes to 60 minutes.

The method of the present application also comprises the step of (2) freezing the cryopreservation solution-soaked cell aggregate obtained in step (1) in vapor phase of a liquid nitrogen container having a temperature of - 150°C or less.

In the present specification, the liquid nitrogen container is a container in which liquid nitrogen is filled, and the space of the vapor phase in the container is maintained at -150°C or less. The temperature of the space of the vapor phase is not particularly limited as long as it is -150°C or less. For example, it may be about -160°C, about -170°C, about -180°C, or about -190°C.

A ratio of volume of liquid nitrogen to volume of the space of the vapor phase in the liquid nitrogen container is not particularly limited as long as the temperature of the space of the vapor phase can be maintained at -150°C or less. Specifically, the volume ratio of liquid nitrogen to the space of the vapor phase in the liquid nitrogen container may be about 1:2 to 1:10. Such liquid nitrogen containers are commercially available as G48-6R (TAIYO NIPPON SANSO CORPORATION) and CryoSystem 6000 (MVE).

Alternatively, liquid nitrogen in a liquid nitrogen container can be infiltered into an absorbent such as glass fiber to provide liquid nitrogen vapor phase atmosphere. Such liquid nitrogen containers, called dry sipper type, are commercially available as DR-22DS (TAIYO NIPPON SANSO CORPORATION) and CryoShipper (MVE).

In the method of the present application, the container in which the cryopreservation solution-soaked cell aggregates are filled is placed in the space of the vapor phase in a liquid nitrogen container.

A proportion of volume of the cell aggregates and the cryopreservation solution filled in the container to volume of the vapor phase in the liquid nitrogen container is not particularly limited as long as the temperature of the space of the vapor phase can be maintained at -150°C or less. The proportion can be appropriately adjusted according to the capacity and volume of the liquid nitrogen vapor phase storage to be used, but it is preferably 5% or less. For example, when the volume of the space of the vapor phase is 1500 L, number of the containers (vials) in which 2 mL of the cell aggregates and cryopreservation solution can be filled is about 1 to 35200.

In the method of the present invention, a filling density of the cell aggregates to the cryopreservation solution is 5 to 500 aggregates/mL or 10 to 500 aggregates/mL, preferably 50 to 500 aggregates/mL, and more preferably 50 to 200 aggregates/mL. The shape and material of the container in which the cell aggregates are filled are not particularly limited, and any container that can seal the cell aggregates and cryopreservation solution can be used.

The container in which the composition comprising the cell aggregates and cryopreservation solution frozen by the method of the present application is filled can be preserved in a liquid nitrogen container for a long-term. In other words, the method of the present application may further comprise the step of (3) preserving the container in which the frozen cell aggregate obtained in step (2) is filled in the vapor phase or liquid phase of a liquid nitrogen container, wherein the container can be preserved at -150°C or less for a long-term.

Alternatively, the container in which the composition comprising the cell aggregates and cryopreservation solution frozen by the method of the present application is filled may be transferred to a deep freezer, a program freezer, a proton freezer, a rapid liquid freezer, an electrostatic air rapid freezer, a Cells Alive System (CAS) rapid freezer, a brine rapid freezer, or another liquid nitrogen container for preservation to preserve it at -80°C or less, preferably at -150°C or less for a long-term. For example, the temperature may be about -160°C, about -170°C, about -180°C, or about -190°C.

The preservation period of the "long-term preservation" is not limited, and for example, one week or more, one month or more, six months or more, one year or more, three years or more, or five years or more. There is no upper limit for the preservation period of the long-term preservation, and the preservation period includes, for example, 10 years, 20 years, 30 years, 50 years, 100 years or more.

The frozen cell aggregate can be thawed and used as appropriate. The thawing method is not particularly limited, but it is desirable to perform the thawing at about body temperature in a short period from the viewpoint of the function, activity, and viability of the cells. Specifically, it is desirable to perform the thawing at 30°C to 40°C, preferably at 35°C to 38°C, and more preferably at a temperature around human body temperature, for example, at about 37°C.

The cell aggregate frozen by the method of the present invention can maintain properties equivalent to those of an unfrozen cell aggregate. For example, the cell aggregate frozen by the method of the present invention and then subjected to recovery culture for 7 days after thawing has a marker expression rate equivalent to that of the cell aggregate before freezing. For example, in the case of the cell aggregate including dopamine-producing neuron progenitor cells, examples of markers expressed on the cells include FOXA2, LMX1A, NURR1 and TH. The equivalent marker expression rate means that the difference in numerical values of the percentages of the cells expressing a maker to the total cells between before freezing and after thawing, or after culturing for 7 days after thawing is about 10% or less.

### [Pharmaceutical composition]

The present application further provides a pharmaceutical composition, i.e., a composition (formulation) for transplantation, comprising, as an active ingredient, the cell aggregate frozen or preserved for a long-term by the above method.

The pharmaceutical composition (composition for transplantation) of the present invention is a concept including both a pharmaceutical composition frozen by the method of the present invention and a pharmaceutical composition obtained by thawing the frozen pharmaceutical composition. That is, examples of the pharmaceutical composition (composition for transplantation) of the present invention include a frozen or unfrozen composition comprising the cell aggregate including neural cells and the cryopreservation solution, as well as a composition comprising the cell aggregate including neural cells and a dosing vehicle where the cryopreservation solution has been replaced with the dosing vehicle after thawing.

Examples of the pharmaceutical composition (composition for transplantation) of the present invention include the compositions for transplantation according to [16] to [20] above.

As one embodiment, the present invention encompasses a composition for transplantation wherein number of cells in the composition is 80000 to 5000000 cells/mL, 100000 to 4000000 cells/mL, or 200000 to 2000000 cells/mL, 300000 to 1000000 cells/mL, and the composition comprises FOXA2-positive and LMX1A-positive cells in an amount of 40% or more, preferably 60% or more, 60% or more, 80% or more, 85% or more, or 90% or more of the total cells, and TH-positive and NURR1-positive cells in an amount of 40% or less, 1% to 20%, or 5% to 15% of the total cells.

In one embodiment, the cell aggregate has an equivalent spherical diameter of 150 to 1000 µm, 150 µm to 600 µm, or 300 µm to 500 µm.

In one embodiment, the cell aggregate and the preservation solution have volume of 0.25 mL to 2 mL, 0.5 mL to 1.5 mL, or 0.5 mL to 1 mL.

In one embodiment, the cell aggregate and the preservation solution may be packed in a 0.5 mL to 15 mL, 0.5 mL to 5 mL, or 1 mL to 2 mL container.

As one embodiment, the present invention encompasses a composition for transplantation that can be used without recovery culture after thawing.

As one embodiment, the present invention encompasses the composition for transplantation according to any one of [16] to [20], wherein the composition comprises the cell aggregates in 8 to 192 cell aggregates/mL, the cell aggregates have an average particle size of 150 µm to 1000 µm, and number of cells per container is 80000 to 2400000.

The cell aggregate is useful as a pharmaceutical composition for transplantation for a patient suffering from a disease requiring transplantation of neural cells and can be used as a drug such as a therapeutic drug for a disease accompanied by degeneration, injury, or dysfunction of neural cells. That is, a pharmaceutical composition comprising the cell aggregate of the present invention and a pharmaceutically acceptable carrier is also within the scope of the present invention.

Examples of the disease requiring transplantation of neural cells or the disease accompanied by injury or dysfunction of neural cells include spinal cord injury, motor nerve disease, multiple sclerosis, amyotrophic lateral sclerosis, atrophic lateral sclerosis, Huntington's disease, multiple system atrophy, spinocerebellar degeneration, Alzheimer's disease, Retinitis pigmentosa, age-related macular degeneration, and parkinsonian syndrome (including Parkinson's disease).

One embodiment of the present invention includes a pharmaceutical composition for treating Parkinson's disease, comprising, as an active ingredient, the cell aggregate of the present invention including dopamine-producing neuron progenitor cells and/or dopamine-producing neurons. Number of dopamine-producing neuron progenitor cells and/or dopamine-producing neurons included in the therapeutic agent for Parkinson's disease is not particularly limited as long as the graft can be engrafted after the administration; for example, 1.0 × 10⁴ cells or more can be included per transplantation. In addition, number of cells may be appropriately increased or decreased according to symptoms or body size to prepare the therapeutic agent. Transplantation of the dopamine-producing neuron progenitor cells to a disease site can be performed, for example, by the technique described in Nature Neuroscience, 2, 1137 (1999) or N Engl J Med.; 344: 710 to 9 (2001).

As one embodiment, the pharmaceutical composition (also referred to as the composition for transplantation) of the present invention comprises a cell aggregate including neural cells to be transplanted into a human and a cryopreservation solution. The pharmaceutical composition of the present invention includes both a frozen solid form and a liquid form before freezing or after thawing. The pharmaceutical composition may include an additive used to maintain the survival of cells as appropriate, to such an extent that it will not affect the freezing rate and freezing temperature. Examples of the cryopreservation solution include those described above.

As described below, the pharmaceutical composition or the composition for transplantation of the present invention is used for transplantation after thawing it and removing and replacing the cryopreservation solution with a dosing vehicle injectable to a living body. That is, a composition comprising the thawed cell aggregate and the dosing vehicle is also within the scope of the pharmaceutical composition (also referred to as the composition for transplantation) of the present invention.

### [A method for producing a composition for transplantation]

The pharmaceutical composition (composition for transplantation) according to [16] to [20] above can be produced by the freezing method according to any one of [1] to [14] above. That is, the present invention encompasses a method for producing the above-described pharmaceutical composition (composition for transplantation).

### [Therapeutic method]

One embodiment of the present invention includes a method for treating a disease requiring supplementation of neural cells, which comprises the step of transplanting the cell aggregate of the present invention into a patient suffering from the disease requiring transplantation of neural cells.

In one embodiment of the present invention, the cell aggregate including dopamine-producing neuron progenitor cells and/or dopamine-producing neurons as obtained by the present invention can be administered to a patient with Parkinson's disease as a pharmaceutical composition, specifically as material for transplantation.

Specifically, the administration is performed as follows: the frozen pharmaceutical composition of the present invention comprising the cell aggregate including dopamine-producing neuron progenitor cells and/or dopamine-producing neurons, and the cryopreservation solution is thawed, appropriately suspended in an appropriate medium for transplantation such as physiological saline, and then transplanted into a region of a patient where dopaminergic neurons are insufficient, for example, the corpus striatum. For example, the pharmaceutical composition after thawing may be washed with a vehicle comprising an appropriate carrier, and the cryopreservation solution may be replaced with a transplantation vehicle for suspending the cell aggregate for the transplantation into a human. A thawing temperature is not particularly limited but can be 30°C to 40°C, preferably 35°C to 38°C, and more preferably a temperature around human body temperature, for example, about 37°C as described above.

The cell aggregate included in the pharmaceutical composition (composition for transplantation) of the present invention can be transplanted into a living body by replacing the cryopreservation solution with a dosing vehicle without recovery culture after thawing.

For the carrier used for the transplantation vehicle (dosing vehicle) used for the cell aggregate including dopamine-producing neuron progenitor cells and/or dopamine-producing neurons, any substance known to those skilled in the art can be used, as long as it is a substance used to maintain survival of cells. Specifically, a physiological aqueous solvent (such as physiological saline, a buffer solution, serum-free medium, etc.) can be used. In transplantation medicine, if necessary, the pharmaceutical composition including tissue or cells to be transplanted may be combined with a preservative, a stabilizing agent, a reducing agent, or an isotonizing agent which is commonly used.

For the transplantation, the thawed cell aggregate may be preserved in a vehicle necessary to maintain the viability of each cell aggregate. Examples of the "vehicle necessary to maintain the viability" include medium and a physiological buffer solution; the vehicle is not particularly limited as long as the vehicle allows the cell population including dopamine-producing neuron progenitor cells and/or dopamine-producing neurons to survive, and those skilled in the art can appropriately select such a vehicle. One example of the vehicle is a medium prepared by using a medium usually used for culturing animal cells as a basal medium. Examples of the basal medium include media that can be used for culturing animal cells, such as BME medium, BGJb medium, CMRL 1066 medium, GMEM medium, Improved MEM Zinc Option medium, Neurobasal medium, IMDM medium, Medium 199 medium, Eagle MEM medium, αMEM medium, DMEM medium, F-12 medium, DMEM/F12 medium, IMDM/F12 medium, Ham medium, RPMI 1640 medium, Fischer's medium or mixed media thereof.

Due to the transplantation of the above cell aggregate, the transplanted dopamine-producing neuron progenitor cells and/or dopamine-producing neurons as well as dopamine-producing neuron progenitor cells and/or dopamine-producing neurons that are induced after the transplantation are functionally engrafted in the patient to which the cell aggregate is administered.

The term "engraftment" as used herein means that transplanted cells survive in a living body for a long period of time (e.g., 30 days or more, 60 days or more, or 90 days or more) and adhere and remain in an organ.

The term "functional engraftment" as used herein means a state in which transplanted cells are engrafted to perform their original function in a living body.

The term "functional survival rate" as used herein refers to the percentage of the cells that have achieved functional engraftment in the transplanted cells. The functional survival rate of transplanted dopamine-producing neuron progenitor cells can be determined, for example, by measuring number of TH-positive cells in the graft.

Due to the transplantation of the above cell aggregate, the transplanted cells and the dopamine-producing neuron progenitor cells and/or dopamine-producing neurons induced after the transplantation have a functional survival rate of 0.1% or more, preferably 0.2% or more, more preferably 0.4% or more, even more preferably 0.5% or more, and still more preferably 0.6% or more.

As one embodiment, the present invention encompasses a method for treating a disease requiring regeneration of a dopamine-producing nerve, which comprises following steps of:
(1) thawing the composition for transplantation according to any one of [16] to [20] at 30°C to 40°C, preferably at 37°C ± 3°C; and
(2) transplanting the composition for transplantation obtained in (1) into a corpus striatum region of a patient.

One embodiment of the present invention encompasses the treating method, wherein the cryopreservation solution is replaced with a dosing vehicle without culture after thawing to perform step (2).

Examples of a mammal to undergo the transplantation in the present specification include humans, mice, rats, guinea pigs, hamsters, rabbits, cats, dogs, sheep, pigs, cows, horses, goats, and monkeys, preferably rodents (e.g., mice and rats) or primates (e.g., humans and monkeys), and more preferably humans.

### EXAMPLES

Hereinafter, the present application is described in more detail with reference to Examples but is not limited to the Examples.

### Example 1: Production of cell aggregates

### Maintenance and neural differentiation of human iPS cells

Human iPSCs (1231A3) (Kyoto University) were maintained in StemFit medium (AJINOMOTO CO., INC.) on a 6-well plate coated with iMatrix-511 (Nippi, Inc.). To initiate neuronal differentiation, the iPSCs were incubated with TrypLE select (Invitrogen) for 10 minutes, then dissociated into single cells and seeded with differentiation medium at a density of 5 × 10⁶ cells/well on a 6-well plate coated with iMatrix-511 (Nippi, Inc.). The differentiation medium was medium containing GMEM supplemented with 8% KSR, 0.1 mM MEM non-essential amino acids (all from Invitrogen), sodium pyruvate (Sigma-Aldrich), and 0.1 mM 2-mercaptoethanol. The differentiation medium was replaced every day from the day after seeding until day 12. To efficiently induce the neuronal differentiation, LDN193189 (STEMGENT, INC.) and A83-01 (WAKO CHEMICAL CO., LTD.) were added. To induce floor plate cells, 2 µM Purmorphamine and 100 ng mL⁻¹ FGF8 (WAKO CHEMICAL CO., LTD.) were added on days 1 to 7, and 3 µM CHIR99021 (WAKO CHEMICAL CO., LTD.) was added on days 3 to 12.

### Culture

The cells on day 12 of culture were reseeded at a density of 1.5 × 10⁴ cells/well in neuronal differentiation medium containing Neurobasal medium supplemented with B27 supplement, 2 µM Glutamax-I (all from Invitrogen), 10 ng mL⁻¹ GDNF, 200 mM ascorbic acid, 20 ng mL⁻¹ BDNF (all from WAKO CHEMICAL CO., LTD.) and 400 µM dbcAMP (Sigma-Aldrich) on a low cell adhesion U-shaped 96-well plate (Sumitomo Bakelite Co., Ltd.). The medium was replaced every 3 days, and 30 µM Y-27632 (WAKO CHEMICAL CO., LTD.) was added to the first medium. For prolonged culture, the floating spheres were cultured in the neuronal differentiation medium until day 28 to obtain a cell aggregate.

### Test Example 1: Cell viability assay

The cell aggregates before freezing obtained in Example 1 were soaked in a water bath (37°C) with a dispersion solution for neuronal cells (Wako Pure Chemical Industries, Ltd.: Cat. No. 297-78101) for 10 minutes, and the spheres were dispersed into single cells by pipetting, and number of viable cells was then determined by Countess^{™} automated cell counter (Thermo Fisher Scientific Inc.: Cat. No. C10281).

In addition, the frozen cell aggregates obtained in Examples 2 to 7 and Comparative Example 1 below were soaked in a water bath (37°C) to be thawed for about 2 minutes, and then subjected to recovery culture in neuronal differentiation medium. After one day of the recovery culture, the supernatant was removed, and number of viable cells was determined in the same manner as in the cell aggregate before freezing.

Cell viability (%) after freezing and thawing in Examples 2 to 7 and Comparative Example 1 was calculated by dividing number of viable cells in the spheres after freezing by number of viable cells in the spheres before freezing.

### Test Example 2: Neurite extension assay

The cell aggregates before freezing obtained in Example 1 were seeded at one cell aggregate per well on a 24-well plate coated with iMatrix-511, cultured in neuronal differentiation medium for 5 days, and then fixed with 4% paraformaldehyde. The fixed spheres were stained with PE-labeled anti-PSA-NCAM antibody (Miltenyi Biotec) and visualized using a fluorescence microscope (BZ-9000; Keyence). The area of the neurites extended from the cell aggregates was measured using Photoshop (Adobe systems) and WinRoof (Mitani Corporation).

In addition, the frozen cell aggregates obtained in Examples 3 to 7 below were soaked in a water bath (37°C) to be thawed for about 2 minutes, and then cultured in neuronal differentiation medium for 5 days. The cell aggregates obtained after 5 days of culture were used to measure the area of the neurites by the same method as for the above cell aggregates.

### Example 2: Freezing of cell aggregates

Sixty four cell aggregates on day 28 produced by the method of Example 1 were collected together with the medium in a 15 mL centrifuge tube (IWAKI: Cat. No. 2323-015), and spontaneously precipitated. The supernatant was then removed, and 1.0 mL of cryopreservation solution, Bambanker^{®} DMSO Free (GC LYMPHOTEC Inc.), was added to obtain a cell aggregate suspension. The total amount of the cell aggregate suspension was injected into a sample container (SARSTEDT: Cat. No. 72.687.028S, 1.5 mL screw cap micro tube), and the sample container was placed on ice for 40 minutes and then placed in a cryobox. In order to evaluate the effect of the method for freezing the cell aggregate, the cryobox was delivered into a liquid nitrogen cryopreservation container (TAIYO NIPPON SANSO CORPORATION: Cat. No. DR-430M, vapor phase preservation type), a -80°C deep freezer (Panasonic Corporation: Cat. No. KM-DU73Y1), and a -150°C deep freezer (Panasonic Corporation: Cat. No. MDF-C2156VAN), and frozen to obtain test preparations of the cell aggregate.

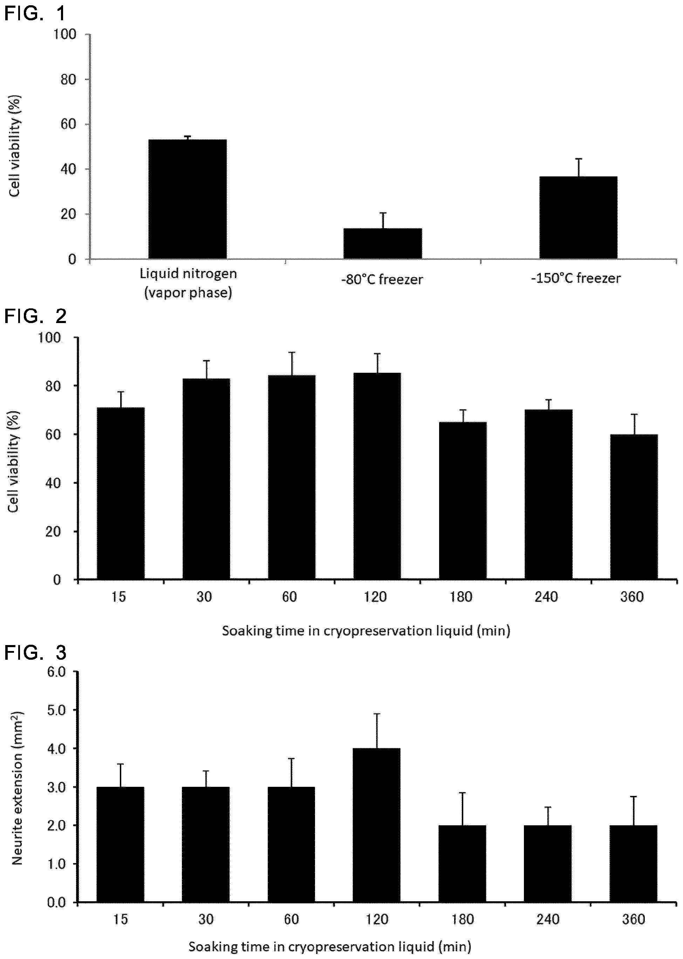

In accordance with the method described in Test Example 1, the cell viability of the test preparations removed from the liquid nitrogen cryopreservation container, the -80°C deep freezer, and the -150°C deep freezer were evaluated (Figure 1).

As a result, the test preparations frozen in the vapor phase space of the liquid nitrogen cryopreservation container had a cell viability of 53.2 ± 1.5% (mean value ± standard deviation, n = 11) after thawing. On the other hand, the test preparations frozen in the -80°C deep freezer (actual temperature in the chamber: -77°C) and the -150°C deep freezer (actual temperature in the chamber: - 145°C) had cell viabilities of 13.7 ± 6.9% (mean value ± standard deviation, n = 11) and 36.8 ± 7.9% (mean value ± standard deviation, n = 11) after thawing, respectively (Figure 1).

Therefore, it was found that the test preparations frozen in the vapor phase space of liquid nitrogen had high cell viability after thawing with significantly suppressed variations among the sample containers.

### Example 3: Effect of the time for soaking a cell aggregate in a cryopreservation solution

Sixty four cell aggregates on day 28 produced by the method of Example 1 were collected together with the medium in a 15 mL centrifuge tube (IWAKI: Cat. No. 2323-015), and spontaneously precipitated. The supernatant was then removed, and 1.0 mL of cryopreservation solution, Bambanker^{®} hRM (GC LYMPHOTEC Inc.), was added to obtain a sphere suspension. In order to evaluate the effect of the time for soaking the cell aggregate in the cryopreservation solution, the total amount of the cell aggregate suspension was injected into a sample container (SARSTEDT: Cat. No. 72.687.028S, 1.5 mL screw cap micro tube), and the sample container was placed on ice for 15 minutes, 30 minutes, 60 minutes, 120 minutes, 180 minutes, 240 minutes, or 360 minutes and then placed in a cryobox. The cryobox was delivered into a liquid nitrogen cryopreservation container (TAIYO NIPPON SANSO CORPORATION: Cat. No. DR-430M, vapor phase preservation type) and frozen to obtain test preparations.

In accordance with the methods described in Test Examples 1 and 2, the cell viability and the neurite extension capacity of the test preparations removed from the liquid nitrogen cryopreservation container were evaluated.

The result is shown in Figures 2 and 3. It was shown that the cell viability and the neurite extension capacity tend to be particularly high when the soaking time in the cryopreservation solution is 15 minutes to 120 minutes.

Therefore, it was considered that the time for soaking the cell aggregates in the cryopreservation solution is particularly preferably 15 minutes to 120 minutes.

### Example 4: Effect of volume of the vapor phase of a liquid nitrogen container

Sixty four cell aggregates on day 28 produced by the method of Example 1 were collected together with the medium in a 15 mL centrifuge tube (IWAKI: Cat. No. 2323-015), and spontaneously precipitated. The supernatant was then removed, and 2 mL of cryopreservation solution, Bambanker^{®} hRM (GC LYMPHOTEC Inc.), was added to obtain a sphere suspension. The total amount of the cell aggregate suspension was transferred to a 2 mL sample container (Nalgene: Cat. No. NL5000-0020), and the sample container was sealed with a cap and placed on ice for 35 minutes. In order to evaluate the effect of a proportion of volume of the cell aggregates and the cryopreservation solution to volume of the vapor phase of the liquid nitrogen container, the present inventors prepared one 25-place cryobox having three of 2 mL sample containers (the proportion of the volume of the cell aggregates and cryopreservation solution filled in the 2 mL sample containers to the volume of the vapor phase of the liquid nitrogen container is 0.3%) and two 25-place cryoboxes each having twenty five of 2 mL sample containers (the proportion of the volume of the cell aggregates and cryopreservation solution filled in the 2 mL sample containers is 5%). These cryoboxes were delivered into the 2L Dewar flask (ISOTHERM: Cat. No. 27B-E) filled with liquid nitrogen for providing liquid nitrogen vapor phase atmosphere, and frozen to obtain test preparations.

In accordance with the methods described in Test Examples 1 and 2, the cell viability and the neurite extension capacity of the test preparations removed from the liquid nitrogen cryopreservation container (2L Dewar flask) were evaluated.

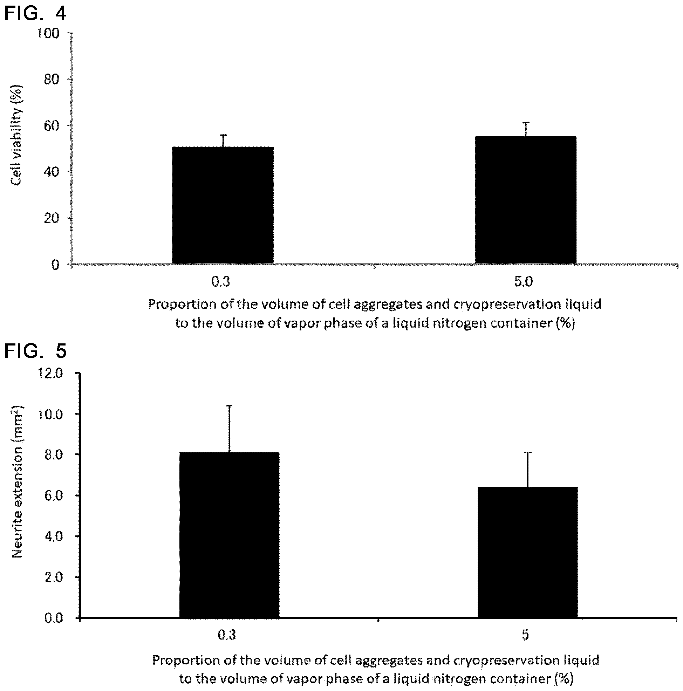

As a result, the cell viabilities and neurite extension capacities were comparable when the proportions of the volume of the cell aggregates and cryopreservation solution filled in the sample containers to the volume of the vapor phase of the liquid nitrogen container were 0.3% and 5% (Figures 4 and 5).

Therefore, it was found that the proportion of the volume of the cell aggregates and cryopreservation solution to the volume of the vapor phase of the liquid nitrogen container has no effect on the cell viability and the neurite extension capacity.

### Example 5: Effect of number of filled cell aggregates

10, 25, 50, 100, 250, or 500 cell aggregates on day 28 produced by the method of Example 1 were collected together with the medium in a 15 mL centrifuge tube (IWAKI: Cat. No. 2323-015), and spontaneously precipitated. The supernatant was then removed, and 1.0 mL of cryopreservation solution, Bambanker^{®} hRM (GC LYMPHOTEC Inc.), was added to obtain a cell aggregate suspension. The total amount of the cell aggregate suspension was transferred to a sample container (1.5 mL SUMILON SuperQuality Slim Tube: Sumitomo Bakelite Co., Ltd.: Cat. No. MS-4702WS), and the sample container was placed on ice for 35 minutes and then placed in a cryobox. This cryobox was delivered into a liquid nitrogen cryopreservation container (TAIYO NIPPON SANSO CORPORATION: Cat. No. DR-430M, vapor phase preservation type) and frozen to obtain a test preparation of the cell aggregates.

In accordance with the methods described in Test Examples 1 and 2, the cell viability and the neurite extension capacity of the test preparations removed from the liquid nitrogen cryopreservation container were evaluated.

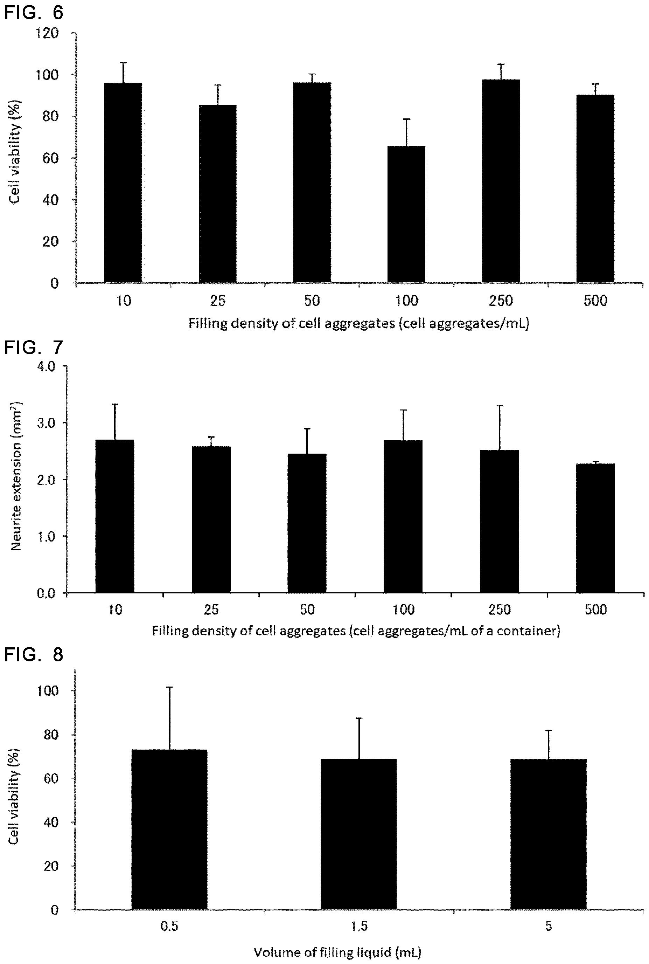

As a result, the cell viabilities and neurite extension capacities were comparable in the test preparations having a filling density in a range of from 10 to 500 cell aggregates/mL, indicating that the filling density of the cell aggregates had no effect on the cell viability and the neurite extension capacity (Figures 6 and 7).

Therefore, it was found that the filling density of the cell aggregates in the sample container has no effect on the cell viability and the neurite extension capacity.

### Example 6: Effect of volume of filled solution per cell aggregate (size of a sample container)

Sixty four cell aggregates on day 28 produced by the method of Example 1 were collected together with the medium in a 15 mL centrifuge tube (IWAKI: Cat. No. 2323-015), and spontaneously precipitated. The supernatant was then removed, and 0.5 mL, 1.5 mL, or 5 mL of cryopreservation solution, Bambanker^{®} hRM (GC LYMPHOTEC Inc.), was added to obtain a sphere suspension. In order to evaluate the effect of volume of filled solution per cell aggregate, the total amounts of 0.5 mL, 1.5 mL, and 5 mL of the cell aggregate suspensions were transferred to a 0.5mL sample container (SARSTEDT: Cat. No. 72.733.001), a 1.5mL sample container (SARSTEDT: Cat. No. 72.687.028S), and a 5mL sample container (Sumitomo Bakelite Co., Ltd.: Cat. No. MS-4605), respectively, and the sample containers were placed on ice for 35 minutes and then placed in cryoboxes. These cryoboxes were delivered into a liquid nitrogen cryopreservation container (TAIYO NIPPON SANSO CORPORATION: Cat. No. DR-430M, vapor phase preservation type) and frozen to obtain test preparations.

In accordance with the methods described in Test Examples 1 and 2, the cell viability and neurite extension capacity of the test preparations removed from the liquid nitrogen cryopreservation container were evaluated.

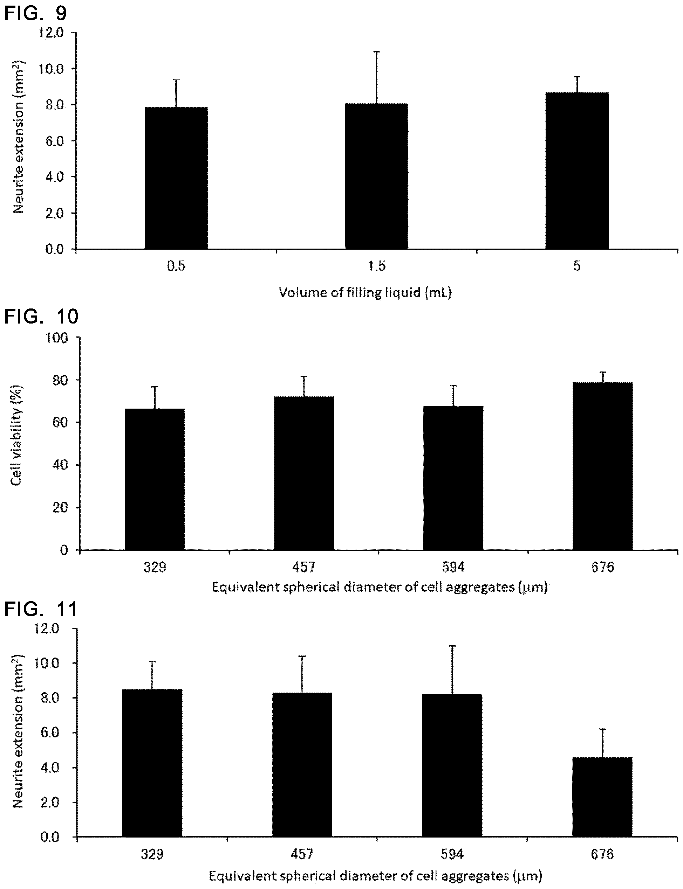

As a result, the cell viabilities and neurite extension capacities were comparable in the 0.5 mL, 1.5 mL, and 5 mL sample containers, indicating that the volume of filled solution per cell aggregate had no effect on the cell viability and the neurite extension capacity (Figures 8 and 9).

### Example 7: Effect of equivalent spherical diameter of a cell aggregate

In the method in Example 1, the cells were reseeded in the neuronal differentiation medium at 0.5 × 10⁴ cells/well, 1.0 × 10⁴ cells/well, 2.0 × 10⁴ cells/well, or 3.0 × 10⁴ cells/well to obtain the cell aggregates having four different sizes (the average equivalent spherical diameter of: 329 µm, 457 µm, 594 µm, or 676 µm) on day 28. These cell aggregates were collected for each size together with the medium in a 15 mL centrifuge tube (IWAKI: Cat. No. 2323-015), and spontaneously precipitated. The supernatants were then removed, and 1.0 mL of cryopreservation solutions, Bambanker^{®} hRM (GC LYMPHOTEC Inc.), was added to obtain cell aggregate suspensions. The total amounts of these cell aggregate suspensions were transferred to their respective sample containers (1.5 mL SUMILON SuperQuality Slim Tube: Sumitomo Bakelite Co., Ltd.: Cat. No. MS-4702WS), and the sample containers were placed on ice for 45 minutes and then placed in a cryobox. This cryobox was delivered into a liquid nitrogen cryopreservation container (TAIYO NIPPON SANSO CORPORATION: Cat. No. DR-430M, vapor phase preservation type) and frozen to obtain test preparations of the cell aggregates.

In accordance with the methods described in Test Examples 1 and 2, the cell viability and neurite extension of the test preparations removed from the liquid nitrogen cryopreservation container were evaluated.

As a result, the cell viabilities were comparable in the cell aggregates having average equivalent spherical diameters of 329 µm, 457 µm, 594 µm, and 676 µm, and the neurite extension capacities tended to be particularly high in the cell aggregates having average equivalent spherical diameters of 329 µm, 457 µm, and 594 µm (Figures 10 and 11).

Therefore, it was considered that the cell aggregate particularly preferably has an equivalent spherical diameter of 329 µm to 594 µm.

### Comparative Example 1

Sixty four cell aggregates on day 28 produced by the method of Example 1 were collected together with the medium in a 15 mL centrifuge tube (IWAKI: Cat. No. 2323-015), and spontaneously precipitated. The supernatant was then removed, and 1.0 mL of cryopreservation solution, Bambanker^{®} DMSO Free (GC LYMPHOTEC Inc.), was added to obtain a cell aggregate suspension. The total amount of the cell aggregate suspension was transferred to a sample container (SARSTEDT: Cat. No. 72.687.028S, 1.5 mL screw cap micro tube), and the sample container was placed on ice for 30 minutes and then placed in a cryobox. The cryobox was soaked in the liquid phase of liquid nitrogen to cool and freeze it to -190°C, and then delivered into a liquid nitrogen cryopreservation container (TAIYO NIPPON SANSO CORPORATION: Cat. No. DR-430M, vapor phase preservation type) for preservation to obtain a test preparation.

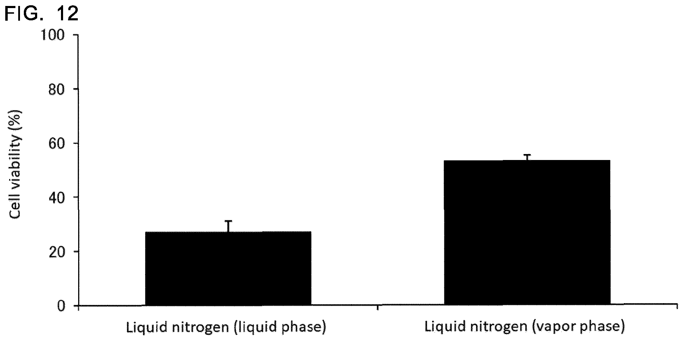

As a result, the cell viability was significantly lower in the test preparation cooled and frozen to -190°C in the liquid phase of liquid nitrogen, compared to that frozen in the vapor phase (see Example 2) (Figure 12).

## Claims

1. A method for freezing a cell aggregate including neural cells and having a three-dimensional structure, which comprises following steps (1) and (2):
(1) soaking the cell aggregate including neural cells in a cryopreservation solution at 0°C to 30°C prior to freezing to prepare a cryopreservation solution-soaked cell aggregate; and
(2) freezing the cryopreservation solution-soaked cell aggregate in vapor phase of a liquid nitrogen container having a temperature of -150°C or less.

2. The method according to claim 1, wherein the cell aggregate is soaked in the cryopreservation solution for 15 minutes to 360 minutes in step (1).

3. The method according to claim 1 or 2, wherein a proportion of volume of the cell aggregate and the cryopreservation solution contained in the liquid nitrogen container to volume of the vapor phase of the liquid nitrogen container is 5% or less.

4. The method according to any one of claims 1 to 3, wherein a filling density of the cell aggregate relative to the preservation solution is 50 to 500 cell aggregates/mL.

5. The method according to claim 4, wherein size of the container containing the cell aggregate and the cryopreservation solution is 0.5 to 5 mL.

6. The method according to any one of claims 1 to 5, wherein the cell aggregate including neural cells is a cell aggregate including neural cells derived from pluripotent stem cells.

7. The method according to any one of claims 1 to 6, wherein the cell aggregate including neural cells comprises cells that are positive for at least one of FOXA2, TH, and NURR1.

8. The method according to claim 7, wherein the cell aggregate including neural cells comprises cells positive for FOXA2 and LMX1A.

9. The method according to claim 7, wherein the cell aggregate including neural cells comprises cells positive for FOXA2, TH, and NURR1.

10. The method according to any one of claims 1 to 9, wherein the neural cells are dopamine-producing neurons or progenitor cells thereof.

11. The method according to any one of claims 1 to 10, wherein the cell aggregate includes 60% or more of dopamine-producing neuron progenitor cells.

12. The method according to any one of claims 1 to 11, wherein the cell aggregate including neural cells is a cell aggregate having an equivalent spherical diameter of 150 µm to 1000 µm.

13. The method according to claim 12, wherein the cell aggregate includes 500 to 150000 cells.

14. The method according to any one of claims 1 to 13, wherein number of cells contained in the cryopreservation solution is 80000 to 5000000 cells/mL.

15. A method for preserving a cell aggregate including neural cells for a long-term, wherein the method comprises storing a container containing the cell aggregate obtained by the method according to any one of claims 1 to 14 in a vapor phase or a liquid phase of the liquid nitrogen container.

16. A composition for transplantation, wherein the composition comprises, as an active ingredient, the cell aggregate obtained by the method according to any one of claims 1 to 14.

17. The composition for transplantation according to claim 16, wherein the composition comprises: a cell aggregate including 60% or more of dopamine-producing neuron progenitor cells and having an equivalent spherical diameter of 150 µm to 1000 µm; and a cryopreservation solution, and wherein the composition can be used without recovery culture after thawing.

18. The composition for transplantation according to claim 16 or 17, wherein number of cells in the composition is 80000 to 5000000 cells/mL.

19. The composition for transplantation according to any one of claims 16 to 18, wherein the composition comprises 10 to 500 cell aggregates/mL.

20. The composition for transplantation according to any one of claims 16 to 19, wherein the cell aggregate and the cryopreservation solution are filled in a 0.5 mL to 15 mL container.

21. A method for producing a composition for transplantation comprising dopamine-producing neuron progenitor cells as an active ingredient, which comprises:
freezing a cell aggregate by the method according to any one of claims 1 to 14, wherein the number of cells in the composition is 80000 to 5000000 cells/mL, and the cell aggregate comprises 60% or more of the dopamine-producing neuron progenitor cells and has an equivalent spherical diameter of 150 µm to 1000 µm.

22. The method for producing the composition for transplantation according to claim 21, wherein the cell aggregate and the cryopreservation solution are filled in a 0.5 mL to 15 mL container.

23. A method for treating a disease requiring regeneration of a dopamine nerve, which comprises following steps of:
(1) thawing the composition for transplantation according to any one of claims 16 to 20 at 30°C to 40°C; and
(2) transplanting the composition for transplantation obtained in (1) into a corpus striatum region of a patient.

24. The method according to claim 23, wherein the cryopreservation solution is replaced with a dosing vehicle without culture after the thawing in (1) to perform step (2).
